(19) Europäisches Patentamt
European Patent Office
Office européen des brevets

(11) Veröffentlichungsnummer: **0 431 425 A2**

(12) **EUROPÄISCHE PATENTANMELDUNG**

(21) Anmeldenummer: **90122435.2**

(22) Anmeldetag: **24.11.90**

(51) Int. Cl.[5]: **C09B 7/00**, C07D 491/10, C07D 471/00, //B41M5/145, B41M5/30,(C07D491/10,311:00, 209:00),(C07D471/10,221:00, 209:00)

(30) Priorität: **07.12.89 DE 3940480**

(43) Veröffentlichungstag der Anmeldung: **12.06.91 Patentblatt 91/24**

(84) Benannte Vertragsstaaten: **CH DE FR GB LI**

(71) Anmelder: **BAYER AG**
**W-5090 Leverkusen 1 Bayerwerk(DE)**

(72) Erfinder: **Meisel, Karlheinrich, Dr.**
**Engstenberger Höhe 11**
**W-5068 Odenthal-Osenau(DE)**
Erfinder: **Psaar, Hubertus, Dr.**
**Paul-Klee-Strasse 21**
**W-5090 Leverkusen 1(DE)**

(54) **Chromogene Enaminverbindungen, ihre Herstellung und Verwendung als Farbbildner.**

(57)

Verbindungen der Formel

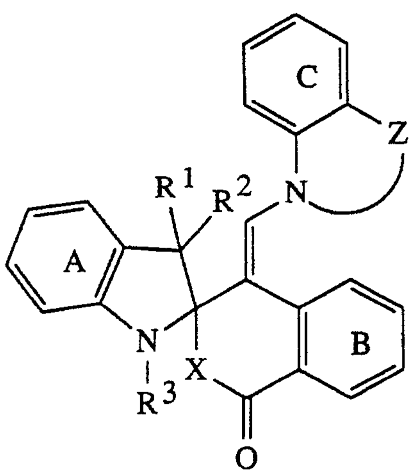

I,

worin

$R^1/R^2$ Alkyl, Cycloalkyl, Aralkyl oder Alkylen,
$R^3$ Alkyl, Cycloalkyl oder Aralkyl,
Z Alkylen,
X 0 oder $NR^4$ und

EP 0 431 425 A2

$R^4$ KW-Rest oder Aryl bedeuten,

eignen sich hervorragend als Farbbildner in Aufzeichnungsmaterialien auf der Basis saurer Entwickler. Man erhält gelbe oder orange Farbtöne, die ausgezeichnet sublimations-und lichtecht sind.

# CHROMOGENE ENAMINVERBINDUNGEN, IHRE HERSTELLUNG UND VERWENDUNG ALS FARBBILDNER

Die vorliegende Erfindung betrifft chromogene Enaminverbindungen, Verfahren zu ihrer Herstellung und ihrer Verwendung in druck- und wärmeempfindlichen Aufzeichnungsmaterialien.

Die neuen Verbindungen entsprechen der allgemeinen Formel

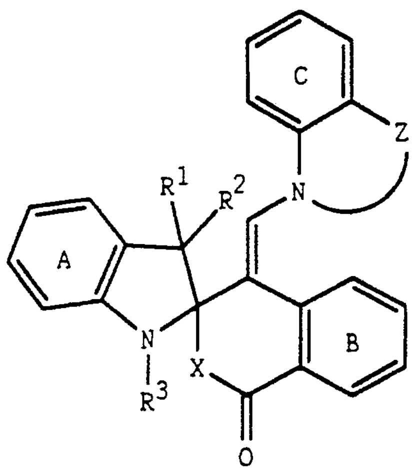

I,

worin

| | |
|---|---|
| $R^1$ und $R^2$ | unabhängig voneinander Alkyl, Cycloalkyl, Aralkyl oder zusammen eine Alkylenbrücke, |
| $R^3$ | Alkyl, Cycloalkyl oder Aralkyl, |
| Z | eine Alkylenbrücke, die ggf. weiter substi tuiert sein kann oder zusammen mit dem Stickstoff und einem weiteren Heteroatom einen 5- oder 6-Ring bildet, und |
| X | Sauerstoff oder $NR^4$, |
| $R^4$ | Wasserstoff, Alkyl, Aryl, Aralkyl, Alkylcarbonyl, Aralkylcarbonyl, Alkylsulfonyl, Aralkylsulfonyl oder Arylsulfonyl bedeuten und |

die Ringe A, B und C sowie die genannten Reste ihrerseits in der Farbstoffchemie übliche nichtionische Substituenten tragen können.

In der Farbstoffchemie übliche nichtionische Substituenten sind z.B. Halogen, Hydroxy, Alkoxy, Aryloxy, Aralkoxy, Heteryloxy, Aryl, Heteryl, Alkylmercapto, Arylmercapto, Aralkylmercapto, Alkylsulfonyl, Cyan, Carbamoyl, Alkoxycarbonyl, Amino, das durch 1 oder 2 Alkyl-, Aryl- oder Aralkylgruppen substituiert sein kann oder dessen Substituenten ringgeschlossen sein können, Alkenyloxy, Alkylcarbonyloxy, Arylcarbonyloxy und als Substituenten der Ringe außerdem Alkyl, Aralkyl, Nitro oder Arylvinyl.

Bevorzugt stehen Alkyl für $C_1$-$C_{30}$-Alkyl, insbesondere für $C_1$-$C_{12}$-Alkyl und ganz besonders für $C_1$-$C_4$-Alkyl, und Alkenyl für $C_2$-$C_5$-Alkenyl.

Geeignete Alkylenbrücken $R^1/R^2$ weisen 5- oder 6 C-Atome auf, während die Alkylenbrücken Z vorzugsweise 2- bis 3-C-Atome in der Kette enthalten.

Unter Halogen ist Fluor, Brom und insbesondere Chlor zu verstehen.

Die Alkylreste und die Alkylreste in Alkoxy-, Alkylthio-, Dialkylamino-, Alkanoylamino-, Alkylsulfonyl- und Alkoxycarbonylgruppen können verzweigt und beispielsweise durch Fluor, Chlor, $C_1$-$C_4$-Alkoxy, Cyano oder $C_1$-$C_4$-Alkoxycarbonyl substituiert sein. Besondere Beispiele sind Methyl, Ethyl, Propyl, 2-Propyl, 2,2-Dimethyl-propyl, 2-Butyl, 1-Hexyl, 1-Octyl, 1-Dodecyl, 1-Tetradecyl, 1-Hexadecyl, 1-Octadecyl, 2-Bornylmethyl, 2-Chlorethyl, 2-Cyanoethyl, 2-Methoxyethyl, 2-Ethoxycarbonylethyl, Trifluormethyl.

Insbesondere werden unter Cycloalkyl Cyclohexyl, unter Aryl Phenyl und Naphthyl, unter Aralkyl Benzyl und Phenethyl, unter Heteryl Pyridyl, Pyrimidyl, Pyrazinyl, Triazinyl, Imidazolyl, Oxazolyl, Thiazolyl, Triazolyl, Thiadiazolyl oder Tetrazolyl, die benzanelliert sein können, und unter Hetaralkyl die genannten Ringe oder Ringsysteme, die über Methylen oder Ethylen an Stickstoff gebunden sind, verstanden. Die Ringe können durch nichtionische Substituenten, insbesondere durch $C_1$-$C_4$-Alkyl, $C_1$-$C_4$-Alkoxy, Cyan, Nitro oder Halogen substituiert sein.

Besondere Beispiele für substituierte Phenylreste sind 2-, 3- oder 4-Tolyl, 2-, 3- oder 4-Anisyl, 2-, 3- oder 4-Chlor-phenyl, 2-, 3- oder 4-Nitro-phenyl, 2-, 3- oder 4-Cyano-phenyl, 2-, 3- oder 4-Ethoxycarbonyl-phenyl, 2-, 3- oder 4-Methoxysulfonyl-phenyl, 2-, 3- oder 4-Trifluormethyl-phenyl, 2,3-Dinitro-phenyl, 3,4-Dimethyl-phenyl, 2-Chlor-4-nitro-phenyl, 3-Chlor-4-nitro-phenyl, 5-Chlor-2-methyl-4-nitro-phenyl, 4-Chlor-3-methyl-phenyl, 3-Chlor-4-methoxy-phenyl, 3-Chlor-4-trifluormethyl-phenyl, 3,4-Dicyano-phenyl, 2,5-Dichlor-4-cyano-phenyl, 2-Methyl-1-naphthyl.

Von besonderem Interesse sind Verbindungen der allgemeinen Formel

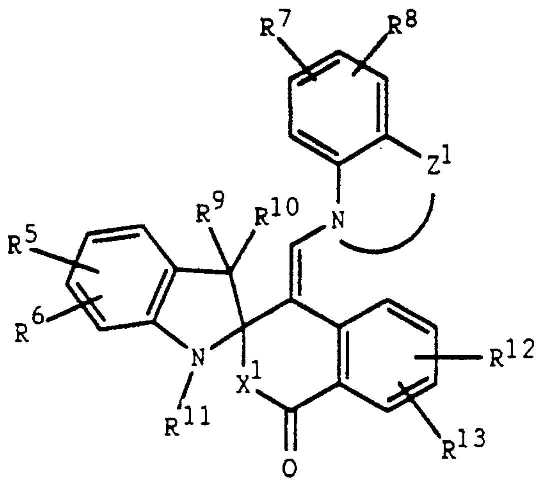

II,

worin - unabhängig voneinander -

| | |
|---|---|
| $R^5$,$R^6$,$R^7$,$R^8$,$R^{12}$,$R^{13}$ | Wasserstoff, $C_1$-$C_{18}$-Alkyl, das ggf. durch Hydroxy, $C_1$-$C_8$-Alkoxy, Cyano, Carbonamido, Chlor oder Brom substituiert sein kann, Phenyl, das ggf. durch Halogen, $C_1$-$C_4$-Alkyl, $C_1$-$C_4$-Alkoxy substituiert sein kann, Phenyl-$C_1$-$C_4$-alkyl, Halogen, $C_1$-$C_{18}$-Alkoxy, Phenoxy, Carboxy, $C_1$-$C_{18}$-Alkoxycarbonyl, $C_1$-$C_{18}$-Alkylsulfonyl, Cyano, Hydroxyl, $C_1$-$C_{18}$-Alkylcarbonyl, $C_1$-$C_{18}$-Alkylcarbonyloxy, $C_1$-$C_{18}$-Alkylamino, $C_1$-$C_{18}$-Dialkylamino, $C_1$-$C_{18}$-N-Alkyl-N-phenylamino, Phenylamino, $C_1$-$C_{18}$-Alkylcarbonylamino, Cyano, Nitro oder |
| $R^5$ u. $R^6$ sowie $R^7$ u. $R^8$ | einen weiteren anellierten Benzolring, |
| $R^9$ und $R^{10}$ | $C_1$-$C_{18}$-Alkyl, $C_5$- oder $C_6$-Cycloalkyl, Benzyl oder zusammen $C_5$- oder $C_6$-Alkylen, die ggf. weiter substituiert sein können, |
| $R^{11}$ | $C_1$-$C_{18}$-Alkyl, Benzyl, Phenethyl, die ggf. weiter substituiert sein können, |
| $Z^1$ | eine $C_2$- oder $C_3$-Alkylenbrücke, die ggf. weiter substituiert sein kann, oder zusammen mit dem Stickstoff und einem weiteren Heteroatom einen 5- oder 6-Ring bildet, |
| $X^1$ | Sauerstoff oder $NR^{14}$ bedeuten, wobei |
| $R^{14}$ | für Wasserstoff, $C_1$-$C_{18}$-Alkyl, Phenyl, $C_1$-$C_4$-Aralkyl, $C_1$-$C_{18}$-Alkylcarbonyl, $C_1$-$C_4$-Aralkylcarbonyl, $C_1$-$C_{18}$-Alkylsulfonyl, $C_1$-$C_4$-Aralkylsulfonyl, die ggf. weiter substituiert sein können, steht. |

Selbstverständlich wird die Auswahl der zahlreichen Substituenten so vorgenommen, daß möglichst preiswerte und anwendungstechnisch optimal wirksame Produkte entstehen. So ist es beispielsweise nicht empfehlenswert, daß in einem Farbbildnermolekül mehr als 2 langkettige (d.h. mehr als 4 C-Atome aufweisende) Alkylreste enthalten sind. Es genügt vielmehr, die Kettenlänge so zu wählen, daß ein im Anwendungsmedium gut lösliches Produkt entsteht.

Von besonderem Interesse sind auch Verbindungen der allgemeinen Formel

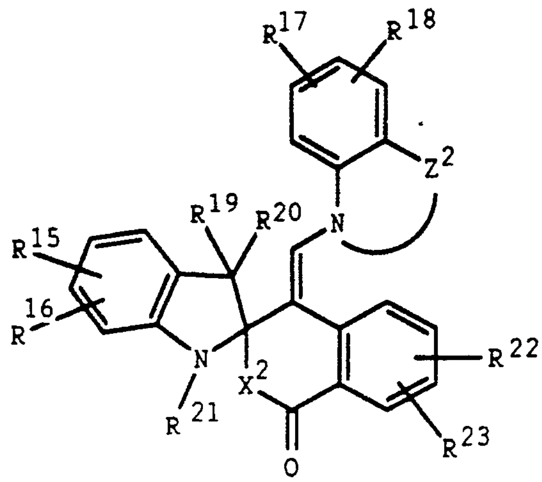

III,

worin - unabhängig voneinander -

| | |
|---|---|
| $R^{15}$,$R^{16}$,$R^{17}$,$R^{18}$ | Wasserstoff, $C_1$-$C_8$-Alkyl, Phenyl, Chlor, Brom, $C_1$-$C_4$-Alkoxycarbonyl, $C_1$-$C_4$-Alkoxy, Cyano oder Nitro, |
| $R^{19}$ und $R^{20}$ | $C_1$-$C_4$-Alkyl, $C_5$- oder $C_6$-Cycloalkyl, Benzyl oder zusammen eine $C_5$- oder $C_6$-Alkylenbrücke, |
| $R^{21}$ | $C_1$-$C_8$-Alkyl, Benzyl, Phenethyl, Cyanoethyl, Amidoethyl, $C_1$-$C_4$-Alkoxyethyl, $C_1$-$C_4$-Halogenalkyl oder $C_1$-$C_4$-Hydroxyalkyl, |
| $Z^2$ | eine $C_2$- oder $C_3$-Alkylenbrücke, die ggf. durch Methylgruppen substituiert sein kann, oder |
| $Z^2$ | zusammen mit dem Stickstoff und einem weiteren NH- oder 0-Atom einen 5- oder 6-Ring bildet, der ggf. durch $C_1$-$C_4$-Alkylgruppen, Halogen oder $C_1$-$C_4$-Alkoxygruppen substituiert sein kann, |
| $R^{22}$ und $R^{23}$ | Wasserstoff, $C_1$-$C_4$-Alkyl, Chlor, Brom oder $C_1$-$C_4$-Alkoxy, |
| $X^2$ | Sauerstoff oder $NR^{24}$ bedeuten, wobei |
| $R^{24}$ | Wasserstoff, $C_1$-$C_{18}$-Alkyl, Phenyl, $C_1$-$C_4$-Alkoxyphenyl, 4-Chlorphenyl, 2-, 3- oder 4-Tolyl, Benzyl, Phenylethyl, $C_1$-$C_{18}$-Alkylcarbonyl, Phenyl-$C_1$-$C_4$-alkylcarbonyl oder $C_1$- $C_{18}$-Alkylsulfonyl sein kann. |

Unter diesen sind vor allem Verbindungen der allgemeinen Formel

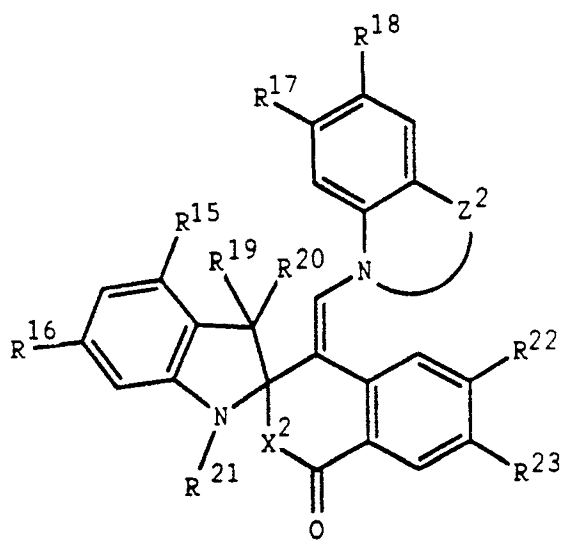

IV,

worin

$R^{15}$ bis $R^{23}$, $X^2$ und $Z^2$ die oben angegebene Bedeutung haben

und Verbindungen der allgemeinen Formel

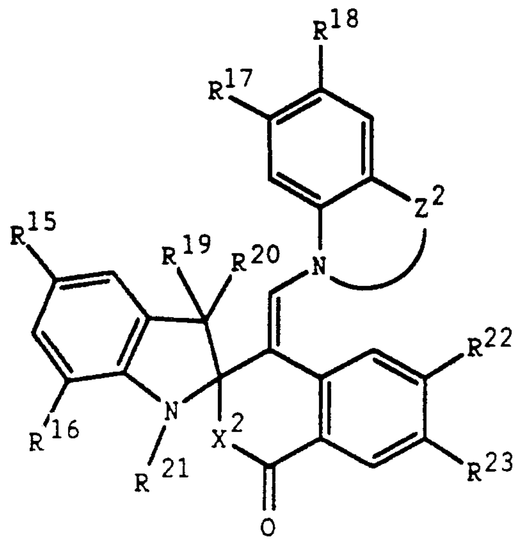

V,

worin

$R^{15}$ bis $R^{23}$, $X^2$ und $Z^2$ die oben angegebene Bedeutung haben

von besonderem Interesse.

Von ganz besonderem Interesse sind Verbindungen der allgemeinen Formel

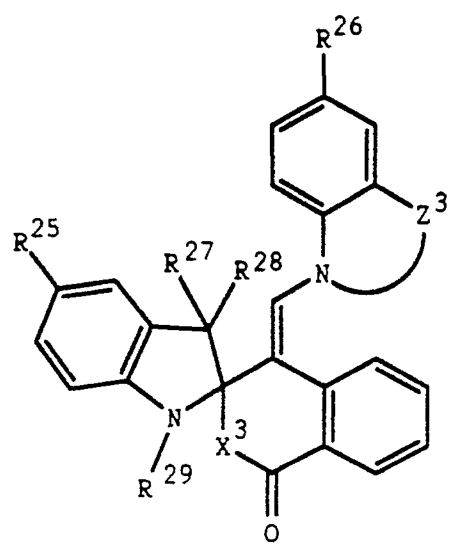

VI,

worin - unabhängig voneinander -

- $R^{25}$ und $R^{26}$ Wasserstoff, $C_1$-$C_4$-Alkyl, $C_1$-$C_4$-Alkoxy, Chlor, Cyano, Nitro oder $C_1$-$C_4$-Alkoxycarbonyl,
- $X^3$ Sauerstoff oder $NR^{27}$,
- $R^{27}$ Wasserstoff, $C_1$-$C_8$-Alkyl, das geradkettig oder verzweigt sein kann, Benzyl oder Phenethyl bedeuten und
- $Z^3$ für eine -$C_2$- oder -$C_3$-Alkylenbrücke steht, die jeweils durch Methylgruppen substituiert sein kann.

Ein weiterer Gegenstand der Erfindung ist ein Verfahren zur Herstellung von Verbindungen der Formel I, welches dadurch gekennzeichnet ist, daß man Verbindungen der Formel

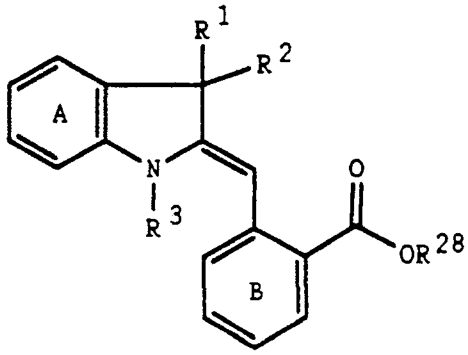

VII,

worin

A, B, $R^1$ bis $R^3$ die oben angegebene Bedeutung haben und

$R^{28}$ für $C_1$-$C_4$-Alkyl, das vorzugsweise durch elektronenanziehende Gruppen, wie z.B. CN oder $COOC_1$-$C_4$-Alkyl substituiert ist, oder ggf. durch $C_1$-$C_4$-Alkyl, Chlor, Nitro, Cyano oder $C_1$-$C_4$-Alkoxy substituiertes Phenyl steht,

mit Verbindungen der allgemeinen Formel

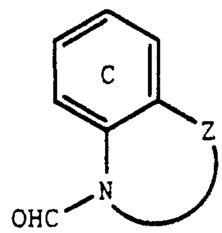

VIII,

worin

C und Z die oben angegebene Bedeutung haben,

in Gegenwart eines wasserentziehenden Mittels kondensiert oder mit Verbindungen der allgemeinen Formel

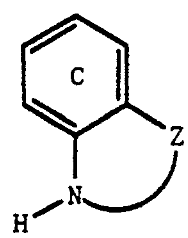

IX,

worin

C und Z die oben angegebene Bedeutung haben,

in Gegenwart eines Orthoameisensäure-$C_1$-$C_4$-alkylesters in einem inerten Lösungsmittel oder einer organischen Säure, insbesondere Essigsäure, umsetzt und die entstehenden Farbstoffe der Formel

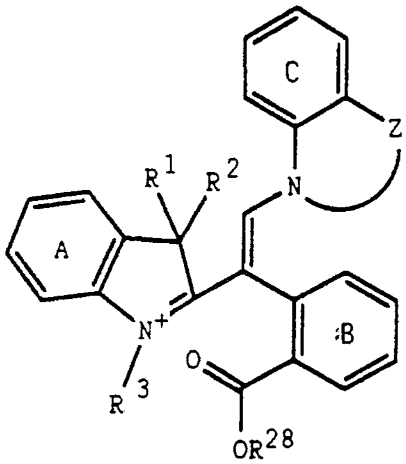

An⁻ X,

worin

| | |
|---|---|
| $R^1$-$R^3$, Z, A, B und C | die oben angegebene Bedeutung haben und |
| $An^-$ | für ein Anion steht, |

zu den Produkten der allgemeinen Formel I cyclisiert, wobei die Cyclisierung entweder in einem wäßrigen oder nichtwäßrigem Medium durch Zugabe einer Base durchgeführt wird.

Anionen $An^-$ sind z.B. $Cl^-$, $Br^-$, $J^-$, $BF_4^-$, $ClO_4^-$, $HSO_4^-$, $SO_4^{2-}$, $SiF_6^{2-}$, $PF_6^-$, $H_2PO_4^-$, $PO_4^{3-}$ oder die Anionen von ggf. durch Halogen, $C_1$-$C_4$-Alkyl, $C_1$-$C_4$-Alkoxy, Hydroxy, Cyano, $C_1$-$C_4$-Alkoxycarbonyl, Nitro oder $C_1$-$C_4$-Alkylsulfonyl substituierten $C_1$-$C_{18}$-Carbonsäuren, $C_2$-$C_{18}$-Dicarbonsäuren, $C_1$-$C_{18}$-Alkylsulfonsäuren, Benzol- oder Naphthalin-mono-oder -dicarbon- oder -sulfonsäuren.

Die Umsetzung der Verbindungen VII und VIII erfolgt üblicherweise mit wasserabspaltenden Reagenzien ohne oder auch mit unter diesen Bedingungen inerten Lösungsmitteln bei Temperaturen zwischen 0° C und dem Siedepunkt des jeweiligen Mediums. Anschließend wird evtl. nach Entfernen der inerten Lösungsmittel auf beispielsweise Wasser oder einen Alkohol ausgetragen.

Wasserabspaltende Reagenzien sind beispielsweise Phosphoroxychlorid, Phosphorpentachlorid, Diphosphorpentoxid, Phosgen, Phosphortrichlorid, Phosphortribromid, Sulfurylchlorid, Thionylchlorid, Oxalylchlorid oder Mischungen hiervon. Vorzugsweise werden Phosphorylchlorid und Phosphoroxychlorid/Diphosphorpentoxid eingesetzt.

Für die Umsetzung von VII und VIII oder VII und IX geeignete inerte Lösungsmittel sind beispielsweise Toluol, Chlorbenzol, Dichlorbenzol, Nitrobenzol, chlorierte aliphatische Kohlenwasserstoffe, wie 1,2-Dichlorethan.

Die Verbindungen der allgemeinen Formel VII erhält man, indem man 2-Methylen-indolinverbindungen der allgemeinen Formel

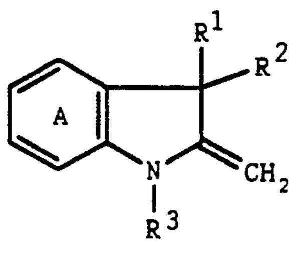

XI

mit o-Halogenbenzoesäuren der allgemeinen Formel

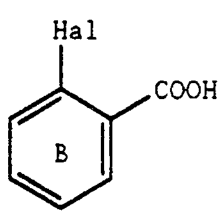

XII

umsetzt, wobei die genannten Reste die oben beschriebene Bedeutung haben und

Hal bevorzugt für Chlor, Brom oder Jod steht,

und die entstehenden Produkte nach literaturbekannten Methoden verestert.

Diese Umsetzung führt man bevorzugt in wäßrigem Medium bei pH-Werten zwischen 4 und 11, insbesondere zwischen 7 und 9, und bei Temperaturen zwischen 60 °C und dem Siedepunkt des Umsetzungsmediums, im allgemeinen bei ~100 °C, durch.

Als Katalysator bewährt sich Cu-Pulver, CuCl und KJ, jeder für sich alleine oder im Gemisch. Die Umsetzungszeiten liegen im allgemeinen zwischen 1 und 48 h.

Als Komponenten der allgemeinen Formel XI kommen z.B. in Frage: 1,3,3-Trimethyl-2-methylen-indolin, 1-Ethyl-3,3-dimethyl-2-methylen-indolin, 1-Cyanoethyl-3,3-dimethyl-2-methylen-indolin, 1-Carbonamidoethyl-3,3-dimethyl-2-methylen-indolin, 1-(2-Hydroxyethyl)-3,3-dimethyl-2-methylen-indolin, 1-(2-Chlorethyl)-3,3-dimethyl-2-methylen-indolin, 1-Octadecyl-3,3-dimethyl-2-methylen-indolin, 1-Octyl-3,3-dimethyl-2-methylen-indolin, 1,3,3-Trimethyl-2-methylen-5-chlor-indolin, 1,3,3-Trimethyl-2-methylen-5-methoxycarbonyl-indolin, 1,3,3-Trimethyl-2-methylen-5-butoxycarbonyl-indolin, 1,3,3-Trimethyl-2-methylen-5-octadecyloxycarbonyl-indolin, 1,3-Dimethyl-3-ethyl-2-methylen-indolin, 1,3-Dimethyl-3-octadecyl-2-methylen-indolin, 1,3-Dimethyl-3-isopropyl-2-methylen-indolin, 1,3,3,4-Tetramethyl-2-methylen-indolin, 1,3,3,5-Tetramethyl-2-methylen-indolin, 1,3,3,6-Tetramethyl-2-methylen-indolin, 1,3,3,7-Tetramethyl-2-methylen-indolin oder Isomerengemische dieser Verbindungen, 1,3,3-Trimethyl-5-octyl-2-methylen-indolin, 1,3,3-Trimethyl-5-dodecyl-2-methylen-indolin, 1,3,3-Trimethyl-5-octadecyl-2-methylen-indolin, 1,3,3-Trimethyl-4,5-benzo-2-methylen-indolin, 1,3,3-Trimethyl-5,6-benzo-2-methylen-indolin, 1,3,3-Trimethyl-6,7-benzo-2-methylen-indolin, 1,3,3-Trimethyl-2-methylen-5-methoxy-indolin, 1,3,3-Trimethyl-2-methylen-5-butoxy-indolin, 1,3,3-Trimethyl-2-methylen-5-octyloxy-indolin, 1,3,3-Trimethyl-2-methylen-5-phenyl-indolin, 1,3,3-Trimethyl-2-methylen-5-phenoxy-indolin, 1,3,3-Trimethyl-2-methylen-5-methylsulfonyl-indolin, 1,3,3-Trimethyl-2-methylen-5-cyano-indolin, 1,3,3-Trimethyl-2-methylen-5-methylcarbonyl-indolin, 1,3,3-Trimethyl-2-methylen-5-octylcarbonyl-indolin, 1,3,3-Trimethyl-2-methylen-5-dimethylamino-indolin, 1,3,3-Trime thyl-2-methylen-5-anilino-indolin, 1,3,3-Trimethyl-2-methylen-5-methylphenylamino-indolin, 1,3,3-Trimethyl-2-methylen-5-acetamino-indolin.

Komponenten der allgemeinen Formel XII können beispielsweise sein: o-Chlor-benzoesäure, 2,4-Dichlor-benzoesäure, 2,4,6-Trichlor-benzoesäure, 2-Chlor-4-methyl-benzoesäure, 2-Chlor-4,6-dimethyl-benzoesäure, 2-Chlor-4-nitro-benzoesäure, 2-Chlor-5-nitro-benzoesäure, 2-Chlor-4,5-dinitro-benzoesäure, 2-Chlor-4,6-dinitro-benzoesäure.

Geeignete Enaminendsysteme sind z.B.:

Indolin, 2-Methyl-indolin, 2,3-Dimethyl-indolin, 2,3,3-Trimethyl-indolin, 2-Phenyl-indolin, Tetrahydrochinolin, 2,2,4-Trimethyl-tetrahydrochinolin, Tetrahydrocarbazol, N-Alkyl-chinoxalin, N-Alkyl-benzimidazolin, Benzoxazolin, die ggf. alle weiter substituiert sein können.

Geeignete Basen zur Cyclisierung des Farbbildners sind beispielsweise Alkalihydroxid, Erdalkalihydroxide, Alkali-oder Erdalkalicarbonate oder -bicarbonate, Amine, wie Ammoniak, Methylamin, Ethylamin, N-Propylamin, Isopropylamin, N-Butylamin, Isobutylamin, sek-Butylamin, tert.-Butylamin, N-Octylamin, Isooctylamin, Octadecylamin oder andere, Benzylamin, Phenylethylamin, Phenyl-butylamin, Anilin, Alkoxyanilin, p-Alkylanilin, Dialkylaminoanilin.

Ein weiterer Gegenstand der Erfindung ist die Verwendung der chromogenen Enaminverbindungen der Formel I oder Mischungen hiervon für druckkopierfähige, thermoreaktive oder elektrochrome Aufzeichnungsmaterialien, wobei das Aufzeichnungsmaterial einen sauren Farbentwickler enthält.

Als saure Entwickler sind insbesondere Tone, saure Oxide oder saure Salze sowie monomere oder polymere Phenole oder Carbonsäuren zu nennen.

Wenn die Farbbildner mit dem sauren Entwickler in Kontakt gebracht werden, ergeben sich intensive gelbe oder orange Farbtöne, die ausgezeichnet sublimations- und lichtecht sind.

Sie sind auch wertvoll im Gemisch mit einem oder mehreren anderen bekannten Farbbildnern, z.B. 3,3-Bis-(amino-phenyl)-phthaliden, 3,3-Bis-(indolyl)-phthaliden, 3-Aminofluoranen, Spirodipyranen, Chromenoindolen, Phenoxazinen, Phenothiazinen, Carbazolylmethanen, 4,4-Diaryldihydrochinazolonen, Benzoxazinen oder anderen Triarylmethanleukofarbstoffen, um grüne, graue oder schwarze Färbungen zu ergeben.

Die Enaminverbindungen der Formel I zeigen sowohl auf phenolischen Unterlagen als auch besonders auf aktivierten Tonen eine gute Farbintensität. Sie eignen sich vor allem als Farbbildner für die Verwendung in einem wärme- oder druckempfindlichen Aufzeichnungsmaterial, das sowohl Kopier- als auch Registriermaterial sein kann. Im allgemeinen zeichnen sie sich durch eine hohe Entwicklungsgeschwindigkeit aus bei gleichzeitig reduzierter Empfindlichkeit der Aufzeichnungsmaterialien gegenüber unbeabsichtigter vorzeitiger Entwicklung.

Die Enaminverbindungen der Formel I zeichnen sich durch gute Lichtechtheit und Klimaalterungsstabilität sowohl im entwickelten als auch unentwickelten Zustand aus.

Ein druckempfindliches Material besteht beispielsweise aus mindestens einem Paar von Blättern, die mindestens einen Farbbildner der Formeln I, gelöst oder dispergiert in einem nichtflüchtigen organischen Lösungsmittel, und einen sauren Entwickler enthalten.

Solche Verfahren und Zubereitungen sind beispielsweise aus den US-Patentschriften 2 800 457, 2 800 458, 2 948 753, 3 096 189 und 3 193 404 und aus den deutschen Offenlegungsschriften 2 555 080 und 2 700 937 bekannt.

Um eine frühzeitige Aktivierung der in dem druckempfindlichen Aufzeichnungsmaterial vorhandenen Farbbildner zu verhindern, werden diese vorzugsweise in Mikrokapseln eingeschlossen, die sich in der Regel durch Druck zerbrechen lassen.

Als Kapselwandmaterialien eignen sich z.B. Gelatine/Gummi arabicum, Polyamide, Polyurethane, Polyharnstoffe, Polysulfonamide, Polyester, Polycarbonate, Polysulfonate, Polyacrylate und Phenol-, Melamin- oder Harnstoff-Formaldehyd-Kondensate, wie sie beispielsweise in M. Gutcho, Capsule Technology and Microencapsulation, Noyes Data Corporation 1972, G. Baster, Microencapsulation, Processses and Applications, Herausgeber J.E. Vandegaar und den deutschen Offenlegungsschriften 2 237 545 und 2 119 933 beschrieben sind.

Bevorzugt werden beim erfindungsgemäßen Verfahren Mikrokapseln verwendet, deren Hüllen aus Polyadditionspro dukten aus Polyisocyanaten und Polyaminen bestehen. Zur Herstellung derartiger Mikrokapseln einzusetzende Isocyanate sind Diisocyanate, Polyisocyanate, Diisocyanate mit Biuretstruktur, durch di- oder trifunktionelle Alkohole modifizierte Polyisocyanate oder andere modifizierte Isocyanate, z.B. solche der Formel:

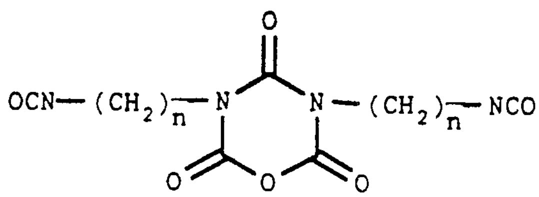

n = 3-6

Zur Umsetzung mit den genannten Isocyanaten geeignete Diamine sind aliphatische primäre oder sekundäre Di- oder Polyamine.

Isocyanate, Amine, Lösungsmittel und ein geeignetes Herstellungsverfahren für solche Mikrokapseln sind beispielsweise in DE-A 32 03 059 beschrieben. Thermoreaktive Aufzeichnungssysteme umfassen z.B. wärmeempfindliche Aufzeichnungs- und Kopiermaterialien und -papiere.

Ein solches Material ist beispielsweise in der DE-A 25 55 080 beschrieben.

Als Entwickler eignen sich die gleichen Elektronenakzeptoren, wie sie in druckempfindlichen Papieren verwendet werden, vorzugsweise phenolische Verbindungen, die beispielsweise in der DE-A 12 51 348 beschrieben sind, sowie Borsäure und organische, vorzugsweise aliphatische Dicarbonsäuren.

Eine weiteres geeignetes thermoreaktives Entwicklungssystem ist in DE-A 33 37 296 beschrieben, bei dem sauer modifizierte Polymerisate, vorzugsweise des Acrylnitrils als Entwickler wirken.

Eine weitere Anwendung der Verbindungen der Formel I-VI ist die Herstellung eines Farbbildes mittels photohärtbarer Mikrokapseln, wie sie z.B. in der DE-A 3 247 488 beschrieben wird.

Die Enaminverbindungen der Formel I bzw. die hieraus durch Ringöffnung gebildeten Farbstoffe der Formel X sind zum Anfärben von Polyacrylnitril, tannierter Baumwolle und anderen sauer modifizierten Fasern, Geweben und Pulvern geeignet.

Beispiel 1

191 g o-Brombenzoesäure, 113 g Wasser und 148 g 1,3,3-Trimethyl-2-methylen-indolin werden mit ~105 ml 30%iger Natronlauge auf pH 8 gestellt, mit 1 g CuCl und 2 g Cu-Pulver versetzt und 12 h auf 100 °C erhitzt. Dabei wird durch kontinuierliche Zugabe von ~89 ml 30%iger Natronlauge am Autotitrator der pH-Wert auf 8 gehalten. Anschließend wird heiß filtriert, mit Schwefelsäure auf pH 6 gestellt und abgesaugt. Der feuchte Niederschlag wird in 1,5 1 Methanol aufgekocht, abgesaugt und getrocknet. Man erhält 184,7 g der Verbindung der Formel:

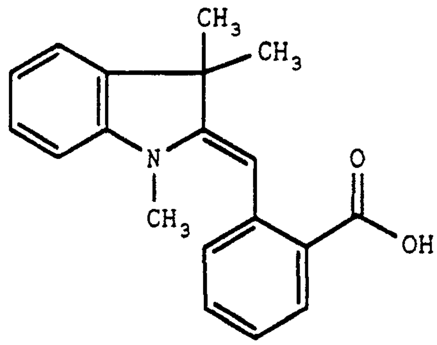

1a

Schmelzpunkt: 132-133° C.

In 800 ml Methylenchlorid werden 141 g der Zwischenverbindung **1a**, 72,5 g p-Nitrophenol und 67,36 g 4-Dimethyl-aminopyridin vorgelegt und mit 160 g Dicyclohexylcarbodiimid versetzt. Man rührt 24 h bei Raumtemperatur und filtriert ab. Die Mutterlauge wird zweimal mit je 1 1 5%iger Essigsäure und 1 1 Wasser ausgeschüttelt und einrotiert. Der Rückstand wird aus Cyclohexan umkristallisiert. Man erhält 188 g der Verbindung der Formel

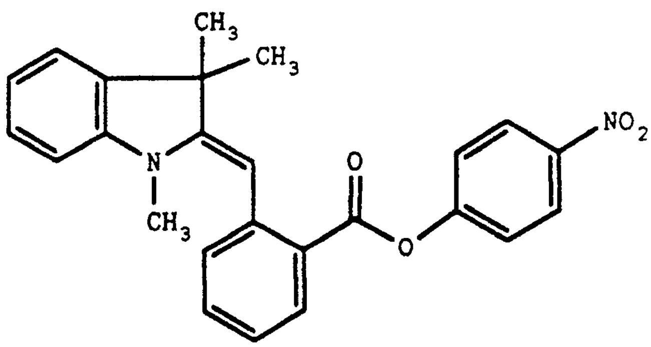

1b

mit dem Schmelzpunkt 125-126° C.

10,32 g des Zwischenproduktes **1b** werden in eine Lösung von 4,025 N-Formyl-2-methyl-indolin in 50 ml Phosphoroxychlorid eingetragen und 12 h bei Raumtemperatur verrührt. Der Ansatz wird auf 500 ml Wasser ausgetragen, wobei nach 24stündigem Nachrühren der Farbstoff der Formel

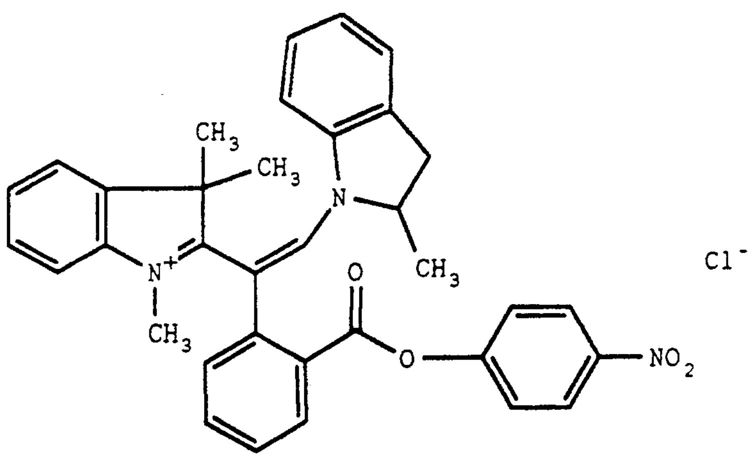

1c

auskristallisiert. Er wird abgesaugt und neutral gewaschen. Der feuchte Niederschlag wird in 100 ml

Dimethyl-formamid gelöst, mit 50%iger Natronlauge versetzt und bei Raumtemperatur gerührt. Nach 1-2 h fällt ein farbloser Niederschlag aus, welcher der folgenden Konstitution entspricht:

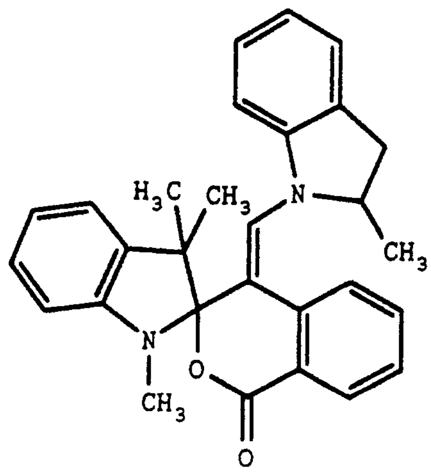

1d

Ausbeute: 2,5 g, $\lambda_{max}$ (in Eisessig): 434 nm.

Beispiel 2

Setzt man die in Beispiel 1 beschriebene Zwischenverbindung **1b** anstelle von N-Formyl-2-methyl-indolin mit 4,025 g N-Formyl-tetrahydrochinolin um und verfährt sonst in gleicher Weise, so gelangt man ebenfalls zu einem wertvollen Farbbildner, der auf Säureton gelb entwickelt ($\lambda_{max}$ (in Eisessig ) = 432 nm).

Beispiel 3

Setzt man die in Beispiel 1 beschriebene Zwischenverbindung **1b** anstelle von N-Formyl-2-methyl-indolin mit 5,075 g N-Formyl-2,2,4-trimethyl-tetrahydrochinolin um und verfährt sonst in gleicher Weise, so gelangt man gleichfalls zu einem wertvollen Farbbildner, der auf Säureton gelb entwickelt ($\lambda_{max}$ ( in Eisessig ) = 431 nm).

Beispiel 4

117,2 g des in Beispiel 1 beschriebenen Zwischenproduktes **1a** werden in 400 ml Chlorbenzol und 192 g 10%iger Natronlauge gelöst, mit 1 g Hexadecyltrimethylammoniumbromid versetzt und bei 40 °C mit 72 g Dimethylsulfat verestert. Man rührt über Nacht, trennt die organische Phase ab, wäscht dreimal mit Wasser und rotiert ein. Man erhält 111,1 g eines gelben Öls, welches der Formel

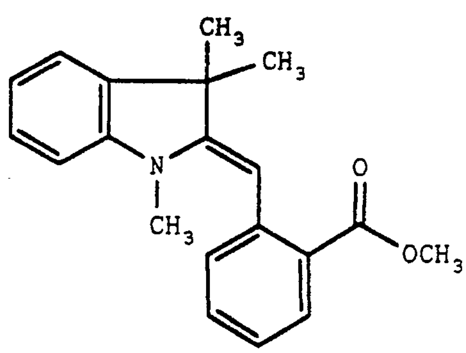

**4a**

entspricht.

Setzt man diese Zwischenverbindung gemäß der in Beispiel 1 beschriebenen Weise weiter um, so gelangt man ebenfalls zu dem Farbbildner des Beispiels 1.

Beispiel 5

29,4 g des in Beispiel 1 beschriebenen Zwischenproduktes **1a** werden mit 21,4 g Diphenylcarbonat und 1 g Natriumcarbonat 5 h auf 140 °C erhitzt. Der abgekühlte Ansatz wird in Chloroform aufgenommen, mit NaOH ausgeschüttelt und im Vakuum einrotiert. Man erhält 22,4 g der Verbindung der Formel

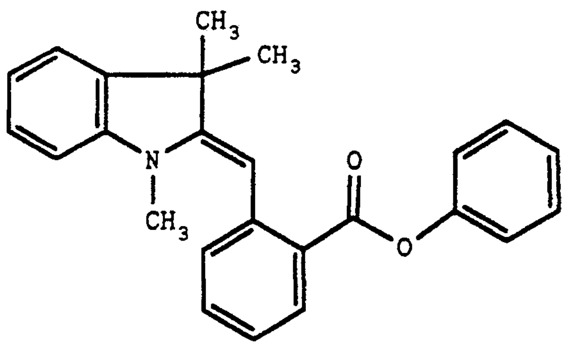

**5a**

Setzt man diese Zwischenverbindung in der in Beispiel 1 angegebenen Weise weiter um, so gelangt man ebenfalls zu dem Farbbildner des Beispiels 1.

Aus der Zwischenverbindung **5a** kann man in analoger Weise auch die Farbbildner der Beispiele 2 und 3 erhalten.

Beispiel 6

Verwendet man anstelle des in Beispiel 1 beschriebenen 1,3,3-Trimethyl-2-methylen-indolins die äquimolekulare Menge 1,3,3-Trimethyl-2-methylen-5-chlor-indolin und verfährt ansonsten weiter nach den Angaben des Beispiels 1, so erhält man einen weiteren wertvollen Farbbildner, der auf Säureton gelb entwickelt ($\lambda_{max}$ (in Eisessig) = 406 nm).

Beispiel 7

Setzt man anstelle des in Beipsiel 1 beschriebenen 1,3,3-Trimethyl-2-methylen-indolins die äquimolekulare Menge 1,3,3-Trimethyl-2-methylen-5-methoxycarbonyl-indolin ein, so erhält man in der 1. Stufe die Zwischenver bindung der Formel:

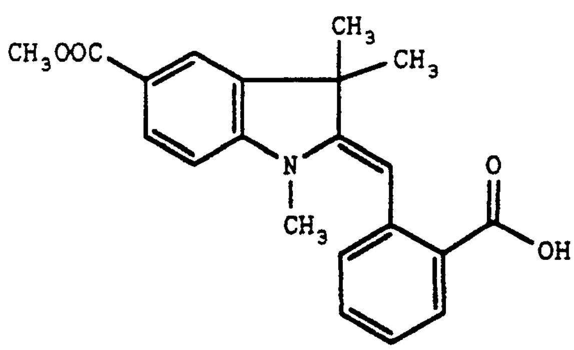

**7a**

Sie läßt sich in Analogie zu Beispiel 1 oder 5 weiter zu einem Farbbildner umsetzen, der auf Säureton gelb entwickelt und in Eisessig $\lambda_{max}$ bei 440 nm zeigt.

Beispiel 8

Zu einem ebenso wertvollen Farbbildner kommt man, wenn man die Zwischenverbindung **7a** des Beispiels 7 in der in Beispiel 1 oder 5 beschriebenen Weise weiter umsetzt, jedoch statt N-Formyl-1,3,3-trimethyl-2-indolin die äquimolare Menge N-Formyl-tetrahydrochinolin einsetzt ($\lambda_{max}$ (in Eisessig) = 438 nm).

Beispiel 9

14 g des in Beispiel 1 erhaltenen Zwischenproduktes **1c** werden in 100 ml n-Butylamin eingetragen und 1 h bei Raumtemperatur verrührt. Der Ansatz wird auf 1 1 Wasser ausgetragen. Das ausgefallene Harz wird in 50 ml Methanol gelöst und auf eine Lösung von 50 ml konz. Natronlauge in 1 1 Wasser ausgetropft. Der farblose Niederschlag wird abgesaugt, neutral gewaschen und getrocknet. Man erhält 6,9 g einer Verbindung der Formel:

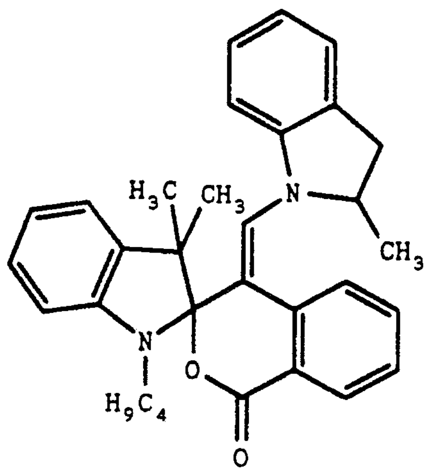

9a

$\lambda_{max}$ (in Eisessig) = 439 nm.

In analoger Weise werden die Verbindungen der allgemeinen Formel 10 hergestellt:

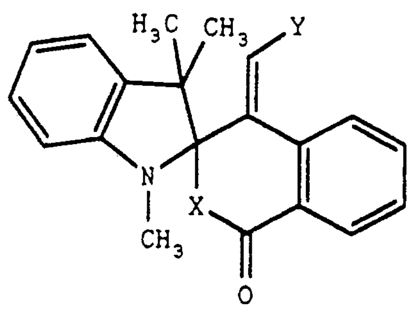

10

Nach der Umsetzung mit Aminen, die nicht mit Wasser mischbar sind, wird zunächst auf verdünnte Salzsäure ausgetragen und der ausgefallene Farbstoff in der in Beispiel 1 beschriebenen Art zum Farbbildner cyclisiert.

| Beispiel | X | Y | $\lambda_{max}$ (Eisessig) |
|---|---|---|---|
| 10 | $i-C_4H_9-N$ | 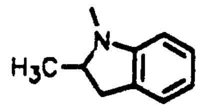 | 438 nm |
| 11 | 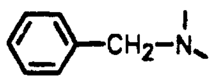 | | 439 nm |
| 12 | 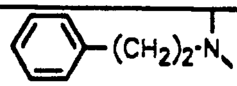 | | 439 nm |
| 13 | 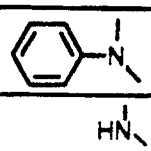 | | 439 nm |
| 14 | $HN$ | 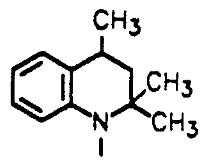 | 426 nm |
| 15 | $H_5C_2-N$ | | 426 nm |
| 16 | $n-C_4H_9-N$ | | 425 nm |
| 17 | $i-C_4H_9-N$ | | 425 nm |
| 18 | 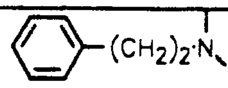 | | 423 nm |
| 19 | 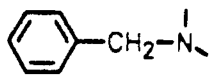 | | 423 nm |
| 20 | $n-C_4H_9-N$ | 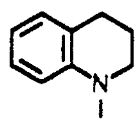 | 433 nm |
| 21 | $i-C_4H_9-N$ | | 433 nm |
| 22 | 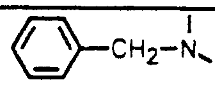 | | 434 nm |
| 23 | 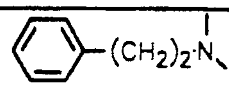 | | 434 nm |
| 24 | $n-C_4H_9-N$ | 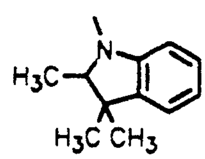 | 438 nm |
| 25 | $i-C_4H_9-N$ | | 436 nm |
| 26 | 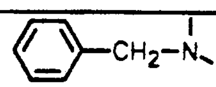 | | 439 nm |
| 27 | 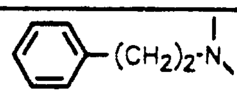 | | 438 nm |

| Beispiel | X | Y | $\lambda_{max}$ (Eisessig) |
|---|---|---|---|
| 28 | 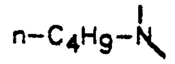 | 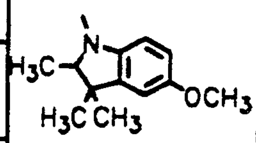 | 451 nm |
| 29 | 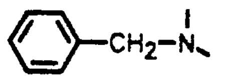 | | 452 nm |
| 30 | 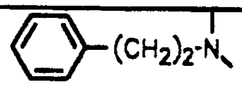 | | 452 nm |
| 31 | 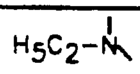 | 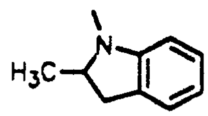 | 439 nm |
| 32 | 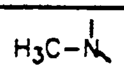 | | 439 nm |
| 33 | 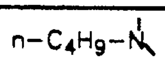 | | 439 nm |
| 34 | 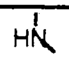 | | 439 nm |
| 35 | 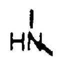 | 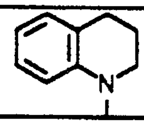 | 433 nm |
| 36 | 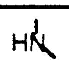 | 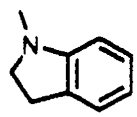 | 436 nm |
| 37 | 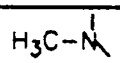 | | 436 nm |
| 38 | 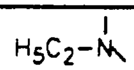 | | 436 nm |

Beispiel 39

Herstellung eines druckempfindlichen kohlefreien Durchschreibepapieres

Eine Lösung von 3 g der Enaminverbindung des Beispiels 1 in 80 g Diisopropylnaphthalin und 17 g Kerosin wird auf an sich bekannte Weise mit Gelatine und Gummiarabicum durch Koazervation mikroverkapselt, mit Stärkelösung vermischt und auf ein Blatt Papier gestrichen. Ein zweites Blatt Papier wird auf der Frontseite mit säureaktiviertem Bentonit als Farbentwickler beschichtet. Das er ste Blatt und das mit Farbentwickler beschichtete Blatt werden mit den Beschichtungen benachbart aufeinandergelegt. Durch Schreiben mit der Hand oder mit der Schreibmaschine auf dem ersten Blatt wird Druck ausgeübt, und es entwickelt sich auf dem mit dem Entwickler beschichteten Blatt eine intensive gelbe Kopie, die ausgezeichnet lichtecht ist.

Wird das mit Mikrokapseln beschichtete Papier mit Tageslicht belichtet und anschließend auf das zweite Blatt geschrieben, so erhält man eine ebenso intensive gelbe Kopie, wie oben beschrieben.

Als Entwicklersubstanzen auf dem handelsüblichen kohlefreien Durchschreibepapier eignen sich gleichermaßen gut z.B. säureaktivierter Bentonit (z.B. Copisil D4A10 der Fa. Südchemie München), p-tert.-Butylphenol-Formaldehyd-Kondensationsprodukte (z.B. UCAR-CKWA 9870 der Fa. Union Carbide Corp.) und p-Alkyl-Zink-Salicylate.

**Ansprüche**

1. Verbindungen der allgemeinen Formel

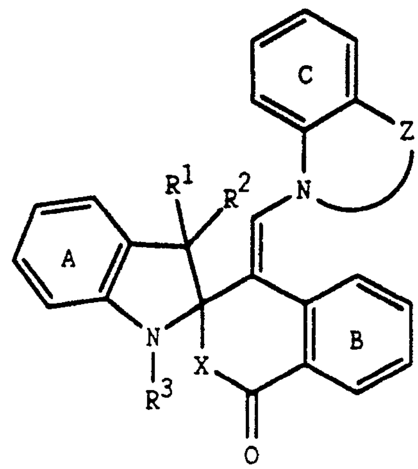

worin

| | |
|---|---|
| $R^1$ und $R^2$ | unabhängig voneinander Alkyl, Cycloalkyl, Aralkyl oder zusammen eine Alkylenbrükke, |
| $R^1$ | Alkyl, Cycloalkyl oder Aralkyl, |
| Z | eine Alkylenbrücke, die ggf. weiter substituiert sein kann oder zusammen mit dem Stickstoff und einem weiteren Heteroatom einen 5- oder 6-Ring bildet, und |
| X | Sauerstoff oder $NR^4$, |
| $R^4$ | Wasserstoff, Alkyl, Aryl, Aralkyl, Alkylcarbonyl, Aralkylcarbonyl, Alkylsulfonyl, Aralkylsulfonyl oder Arylsulfonyl bedeuten und |

die Ringe A, B und C sowie die genannten Reste ihrerseits in der Farbstoffchemie übliche nichtionische Substituenten tragen können,
sowie die daraus durch Ringöffnung ableitbaren kationischen Farbstoffe.

2. Verbindungen der allgemeinen Formel

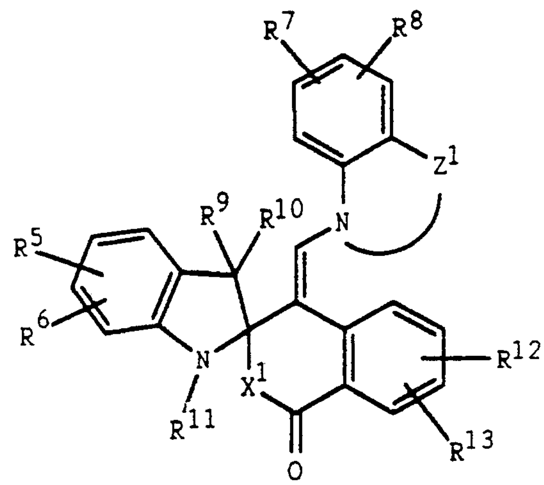

worin - unabhängig voneinander -

| | |
|---|---|
| $R^5$,$R^6$,$R^7$,$R^8$,$R^{12}$,$R^{13}$ | Wasserstoff, $C_1$-$C_{18}$-Alkyl, das ggf. durch Hydroxy, $C_1$- $C_8$-Alkoxy, Cyano, Carbonamido, Chlor oder Brom substituiert sein kann, Phenyl, das ggf. durch Halogen, $C_1$-$C_4$-Alkyl, $C_1$-$C_4$-Alkoxy substituiert sein kann, Phenyl-$C_1$-$C_4$-alkyl, Halogen, $C_1$-$C_{18}$-Alkoxy, Phenoxy, Carboxy, $C_1$-$C_{18}$-Alkoxycarbonyl, $C_1$-$C_{18}$-Alkylsulfonyl, Cyano, Hydroxyl, $C_1$-$C_{18}$-Alkylcarbonyl, $C_1$-$C_{18}$-Alkylcarbonyloxy, $C_1$-$C_{18}$-Alkylamino, $C_1$-$C_{18}$-Dialkylamino, $C_1$-$C_{18}$-N-Alkyl-N-phenylamino, Phenylami no, $C_1$-$C_{18}$-Alkylcarbonylamino, Cyano, Nitro oder |
| $R^5$ u. $R^6$ | sowie $R^7$ u. $R^8$ einen weiteren anellierten Benzolring, |

| | |
|---|---|
| $R^9$ und $R^{10}$ | $C_1$-$C_{18}$-Alkyl, $C_5$- oder $C_6$-Cycloalkyl, Benzyl oder zusammen $C_5$- oder $C_6$-Alkylen, die ggf. weiter substituiert sein können, |
| $R^{11}$ | $C_1$-$C_{18}$-Alkyl, Benzyl, Phenethyl, die ggf. weiter substituiert sein können, |
| $Z^1$ | eine $C_2$- oder $C_3$-Alkylenbrücke, die ggf. weiter substituiert sein kann, oder zusammen mit dem Stickstoff und einem weiteren Heteroatom einen 5- oder 6-Ring bildet, |
| $X^1$ | Sauerstoff oder $NR^{14}$ bedeuten, wobei |
| $R^{14}$ | für Wasserstoff, $C_1$-$C_{18}$-Alkyl, Phenyl, $C_1$-$C_4$-Aralkyl, $C_1$-$C_{18}$-Alkylcarbonyl, $C_1$-$C_4$-Aralkylcarbonyl, $C_1$-$C_{18}$-Alkylsulfonyl, $C_1$-$C_4$-Aralkylsulfonyl, die ggf. weiter substituiert sein können, steht. |

**3.** Verbindungen der allgemeinen Formel

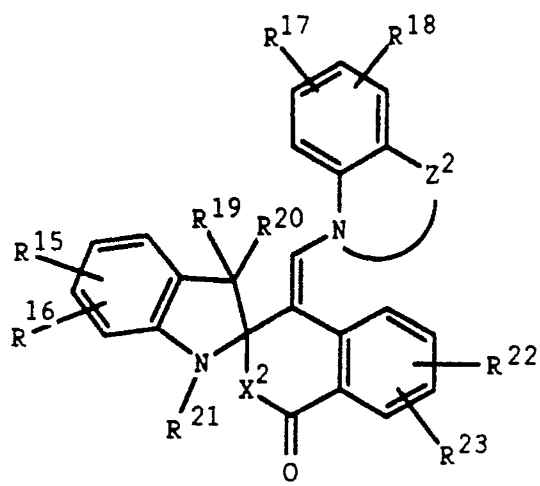

worin - unabhängig voneinander -

| | |
|---|---|
| $R^{15}$,$R^{16}$,$R^{17}$,$R^{18}$ | Wasserstoff, $C_1$-$C_8$-Alkyl, Phenyl, Chlor, Brom, $C_1$-$C_4$-Alkoxycarbonyl, $C_1$-$C_4$-Alkoxy, Cyano oder Nitro, |
| $R^{19}$ und $R^{20}$ | $C_1$-$C_4$-Alkyl, $C_5$- oder $C_6$-Cycloalkyl, Benzyl oder zusammen eine $C_5$- oder $C_6$-Alkylenbrücke, |
| $R^{21}$ | $C_1$-$C_8$-Alkyl, Benzyl, Phenethyl, Cyanoethyl, Amidoethyl, $C_1$-$C_4$-Alkoxyethyl, $C_1$-$C_4$-Halogenalkyl oder $C_1$-$C_4$-Hydroxyalkyl, |
| $Z^2$ | eine $C_2$- oder $C_3$-Alkylenbrücke, die ggf. durch Methylgruppen substituiert sein kann, oder |
| $Z^2$ | zusammen mit dem Stickstoff und einem weiteren NH- oder 0-Atom einen 5- oder 6-Ring bildet, der ggf. $C_1$-$C_4$-Alkylgruppen, Halogen oder $C_1$-$C_4$-Alkoxy-grup pen substituiert sein kann, |
| $R^{22}$ und $R^{23}$ | Wasserstoff, $C_1$-$C_4$-Alkyl, Chlor, Brom oder $C_1$-$C_4$-Alkoxy, |
| $X^2$ | Sauerstoff oder $NR^{24}$ bedeuten, wobei |
| $R^{24}$ | Wasserstoff, $C_1$-$C_{18}$-Alkyl, Phenyl, $C_1$-$C_4$-Alkoxyphenyl, 4-Chlorphenyl, 2-, 3- oder 4-Tolyl, Benzyl, Phenylethyl, $C_1$-$C_{18}$-Alkylcarbonyl, Phenyl-$C_1$-$C_4$-alkylcarbonyl oder $C_1$- $C_{18}$-Alkylsulfonyl sein kann. |

**4.** Verbindungen der allgemeinen Formel

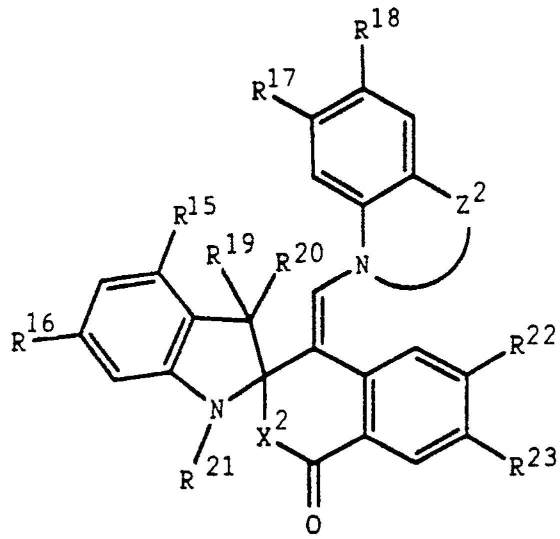

worin
$R^{15}$ bis $R^{23}$, $X^2$ und $Z^2$ die in Anspruch 3 angegebene Bedeutung haben.

**5.** Verbindungen der allgemeinen Formel

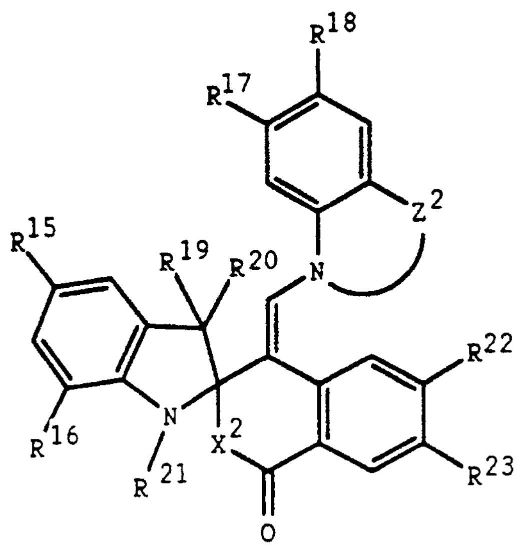

worin
$R^{15}$ bis $R^{23}$, $X^2$ und $Z^2$ die in Anspruch 3 angegebene Bedeutung haben.

**6.** Verbindungen der allgemeinen Formel

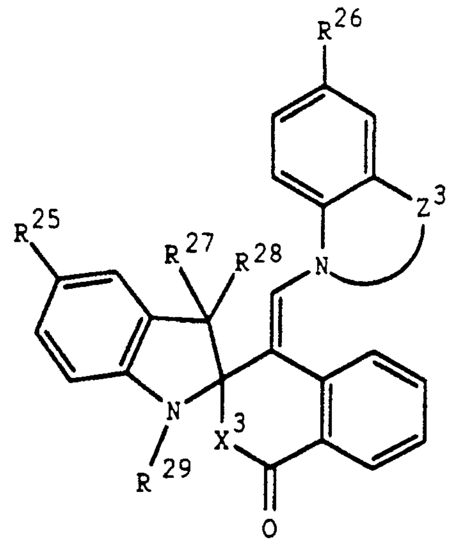

worin - unabhängig voneinander -

| | |
|---|---|
| $R^{25}$ und $R^{26}$ | Wasserstoff, $C_1$-$C_4$-Alkyl, $C_1$-$C_4$-Alkoxy, Chlor, Cyano, Nitro oder $C_1$-$C_4$-Alkoxycarbonyl, |
| $X^3$ | Sauerstoff oder $NR^{27}$, |
| $R^{27}$ | Wasserstoff, $C_1$-$C_8$-Alkyl, das geradkettig oder verzweigt sein kann, Benzyl oder Phenethyl bedeuten und |
| $Z^3$ | für eine -$C_2$- oder -$C_3$-Alkylenbrücke steht, die jeweils durch Methylgruppen substituiert sein kann. |

7. Verbindung der Formel:

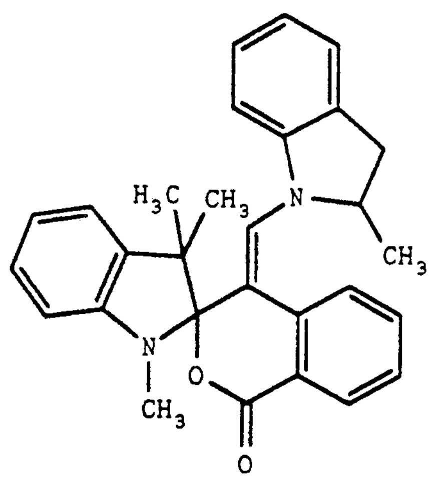

8. Verbindung der Formel:

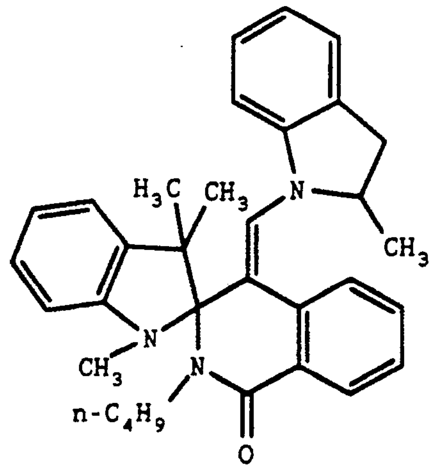

9. Verfahren zur Herstellung von Verbindungen des Anspruchs 1, dadurch gekennzeichnet, daß man Verbindungen der Formel

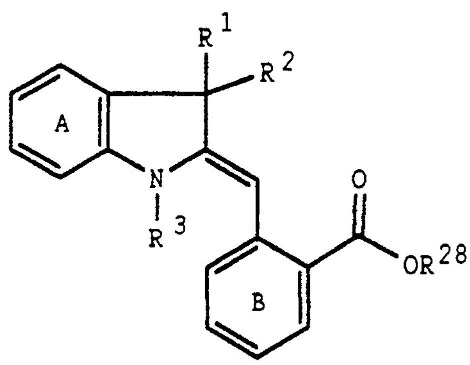

worin

A, B, $R^1$-$R^3$ die in Anspruch 1 angegebene Bedeutung haben und

$R^{28}$ für $C_1$-$C_4$-Alkyl, das vorzugsweise durch elektronenanziehende Gruppen, wie z.B. CN oder $COOC_1$-$C_4$-Alkyl substituiert ist, oder ggf. durch $C_1$-$C_4$-Alkyl, Chlor, Nitro, Cyano oder $C_1$-$C_4$-Alkoxy substituiertes Phenyl steht,

mit Verbindungen der allgemeinen Formel

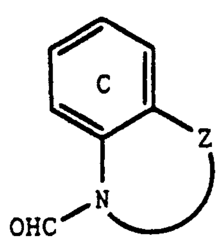

worin

C und Z die in Anspruch 1 angegebene Bedeutung haben,

in Gegenwart eines wasserentziehenden Mittels kondensiert oder mit Verbindungen der allgemeinen Formel

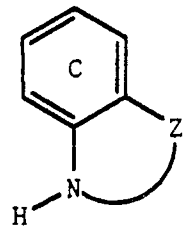

worin

C und Z die in Anspruch 1 angegebene Bedeutung haben,

in Gegenwart eines Orthoameisensäure-$C_1$-$C_4$-alkylesters in einem inerten Lösungsmittel oder einer organischen Säure, insbesondere Essigsäure, umsetzt und die entstehenden Farbstoffe der Formel

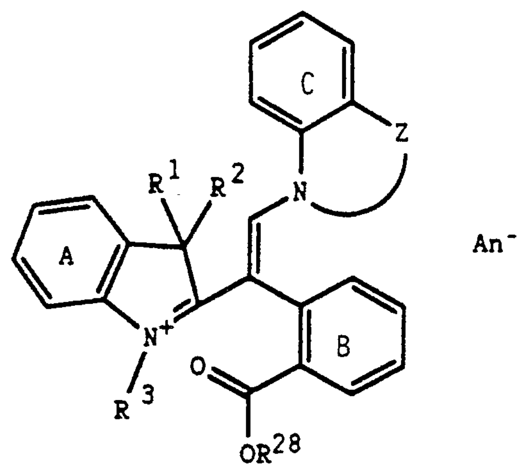

worin

$R^1$-$R^3$, Z, A, B und C die Anspruch 1 angegebene Bedeutung haben und

$An^-$ für ein Anion steht,

zu den Produkten des Anspruchs 1 cyclisiert, wobei die Cyclisierung entweder in einem wäßrigen oder nichtwäßrigem Medium durch Zugabe einer Base durchgeführt wird.

**10.** Druck- oder wärmeempfindliches Aufzeichnungsmaterial, dadurch gekennzeichnet, daß es in seinem Farbreaktantensystem als Farbbildner mindestens eine Enaminverbindung der Ansprüche 1 bis 6 enthält.